# EUROPEAN PATENT APPLICATION

(11) **EP 1 464 316 A2**
(43) Date of publication of application: **06.10.2004**
(21) Application number: 04075661.1
(22) Date of filing: 03.03.2004
(51) Int. Cl.: A61K 7/04, A61K 7/043

(54) **Method for the lengthening and/or reconstruction of a natural nail, gel and nail supporting structure for the application of the method**

(30) Priority: 06.03.2003 IT MI20030408
(71) Applicant: Layla Cosmetics S.r.L., 20138 Milan (IT)
(72) Inventor: Rossetti, Emma, 20122 Milan (IT)
(74) Representative: Martegani, Franco

(57) **Abstract**

Use of a photocuring gel with a low viscosity consisting of a modified acrylate polymer selected from aliphatic diacrylates modified with a monomeric photoionizer, as gel for the lengthening and/or reconstruction of natural nails.

## Description

The present invention relates to a method for the lengthening and/or reconstruction of a natural nail as well as a gel and a nail supporting structure, for the application of the method.

The problem of lengthening and/or reconstructing nails is a strongly felt problem which is becoming increasingly more important for many reasons.

In the first place, for an ever growing number of people who work in direct contact with the public, the necessity of always having impeccable hands, which consequently also implies having nails in a perfect state.

Furthermore, in the case of people who bite their nails as a result of stress and/or nervousness, this problem cannot be solved by means of simple manicure, as the length and/or overall state of the natural nail does not allow a satisfactory result to be obtained.

The known art has tried to solve this problem by the application of false nails or tips (tip refers to a prosthesis which covers only the end part of the natural nail and which can be cut to the desired length and shape) applied by means of suitable adhesives on a base consisting of the natural nail to be treated.

The false nail is then treated directly with varnish/es, whereas in the case of tips further intermediate treatment is necessary. Resins and/or photocuring gels, have been found, which are extremely dense and have a high viscosity which, when applied to tips, create a non-homogeneous and very thick surface. This situation, generated by the use of these very dense gels, consequently requires vigorous filing to ensure that the important thickness created on the surface of the nail, resembles that of the natural nail.

These methods, however, for the reasons indicated below, can only be applied by professionally skilled people, for example in beauty parlours, as direct use on the part of the final user is practically impossible.

Both the complexity of the method itself and the use of products which are difficult and complex to apply, as also the cost of these products and the necessary equipment (such as a UV lamp which is difficult to find on the market), prevent final users from autonomously succeeding in lengthening and/or reconstructing their nails, contrary to what is true for a normal manicure.

The methods according to the known art are therefore extremely complex, require long times for their application and have high costs.

The present invention proposes to overcome the drawbacks present in the known art.

In particular, an object of the present invention relates to the use of a photocuring gel with a low viscosity consisting of a modified acrylate polymer selected from aliphatic diacrylates modified with a monomeric photo-ionizer, as gel for the lengthening and/or reconstruction of natural nails.

A further object of the present invention relates to a method for the lengthening and/or reconstruction of a natural nail which comprises the following phases:
a) preparation of the nail with a mousse pumice (abrasive sponge) and subsequent degreasing treatment;
b) insertion on the end of the natural nail thus cleaned, of a nail supporting structure;
c) application on the nail, positioned in the nail supporting structure, and optionally on the portion of the supporting structure adjacent to the nail end up to the desired length, of a low viscosity photocuring gel consisting of a modified acrylate polymer selected from aliphatic diacrylates modified with a monomeric photo-ionizer;
d) application to the nail thus treated of a UV radiation for a time varying from 2 to 8 minutes, preferably from 5 to 8 minutes;
e) repetition of phases a)-d) for each natural nail to be lengthened and/or reconstructed and optionally phases c)-d) for each touch-up of the nail already treated;
f) removal of the nail supporting structure from the treated nail;
g) treatment of the nail thus prepared with a degreasing compound of the residual part of the gel after hardening;
h) filing of the gel at the end of the nail until the desired form and length are obtained.

A further object of the present invention also relates to a supporting structure for the application of a gel for the lengthening and/or reconstruction of a natural nail, consisting of a tubular element made of a flexible material (for example a plastic material), suitable for containing a finger, comprising a supporting portion at an end of the structure and an opening suitable for completely exposing the natural nail to be treated, in an adjacent position to said supporting portion, said supporting portion having a rounded edge towards the opening, suitable for being positioned between the lower surface of the natural nail to be treated and the underlying finger.

In particular, the use of a low viscosity photocuring gel consisting of a diacrylate of oxybis (methyl-2,1-ethanediyl) is preferred as gel for the lengthening and/or reconstruction of natural nails.

The photocuring gel to be used as gel for the lengthening and/or reconstruction of natural nails, according to the present invention, has a viscosity at 25°C ranging from 3500 to 4500 cps.

Furthermore the photocuring gel to be used as gel for the lengthening and/or reconstruction of natural nails, according to the present invention, can be diluted with a dispersion of nitrocellulose and titanium dioxide in a mixture of solvents, such as, for example, ethyl acetate, butyl acetate and isopropyl alcohol.

The method for the lengthening and/or reconstruction of natural nails, according to the present invention, also envisages that phases c) and d) are effected at least a second time on the same nail, before carrying out phase e).

The method for the lengthening and/or reconstruction of natural nails, according to the present invention, can also comprise the application of a tip to the nail to be treated by means of an adhesive, before effecting phase b).

The method for the lengthening and/or reconstruction of natural nails, according to the present invention, preferably envisages the use of a photocuring gel which is a diacrylates of oxybis(methyl-2,1-ethanediyl), and even more preferably it has a viscosity at 25°C ranging from 3500 to 4500 cps.

Furthermore the method for the lengthening and/or reconstruction of natural nails, according to the present invention, preferably comprises the use of a photocuring gel diluted with a dispersion of nitrocellulose and titanium dioxide in a mixture of solvents, such as, for example, ethyl acetate, butyl acetate and isopropyl alcohol.

The method for the lengthening and/or reconstruction of natural nails, according to the present invention, preferably envisages that the degreasing compound in phase a) is substantially a mixture of methyl methacrylate monomer and toluidine.

Furthermore, the method for the lengthening and/or reconstruction of natural nails, according to the present invention, envisages that the degreasing compound for gel in phase g) is substantially isopropyl alcohol.

The main advantage of the method according to the present invention consists in its simplicity and practicalness, which allow it to be effected in much shorter times with respect to the existing methods, and above all allow it to be effected directly on the part of the final user. This simplicity and practicalness are mainly due to the use of the particular low viscosity photocuring gel and the particular nail supporting structure, also object of the present invention.

A further advantage consists in the possibility of packing the low viscosity photocuring gel to be used for the lengthening and/or reconstruction of natural nails, according to the present invention, in a normal varnish container with the relative applier. Unlike the gel of the known art, it is therefore not necessary to apply the gel by means of brushes ad hoc. The low viscosity photocuring gel to be used for the lengthening and/or reconstruction of natural nails, according to the present invention, is in fact self-leveling and consequently does not require the use of long brushes, or complex and long filing operations on the upper surface of the nail after the application of the gel, these operations, on the other hand, being indispensable with the resins and gels according to the state of the art.

The use of the gel according to the present invention also allows the lengthening and/or reconstruction of natural nails to be effected without the tip, a solution which was not possible with the high viscosity gels previously used.

It also allows, together with the supporting structure, the lengthening and/or reconstruction to be effected starting from a very short natural nail base.

The low viscosity gel according to the present invention can therefore be applied with the same facility and simplicity that a user is accustomed to, when using a normal nail varnish.

Furthermore, the nail supporting structure according to the present invention also solves the drawbacks of the state of the art relating to said supports. The state of the art, in fact, consists in the use of disposable structures, generally made of adhesive paper, to be stuck to the nail, which proved to be difficult and complex to apply. The supporting structure for the application of the method for the lengthening and/or reconstruction of natural nails, according to the present invention, can, on the other hand, be re-used. As it can be simply and immediately applied to the finger, it forms a better support for the low viscosity photocuring gel and therefore allows nails to be lengthened and/or reconstructed in various forms and lengths.

Further advantages and characteristics of the present invention will appear more evident from the following description. It is of an illustrative but non-limiting nature and refers to the enclosed drawings, in which:
Figure 1 shows a perspective view of an illustrative embodiment of the supporting structure for the application of the method for the lengthening and/or reconstruction of a natural nail according to the present invention;
Figure 2 shows a plan view from above of an embodiment of a supporting structure for the application of the method for the lengthening and/or reconstruction of a natural nail according to the present invention;
Figure 3 shows a plan view from below;
Figures 4 and 5 are two views of the end of said structure, respectively; and
Figure 6 is a perspective view illustrating the structure of figures 1-5 applied to the finger of a hand.

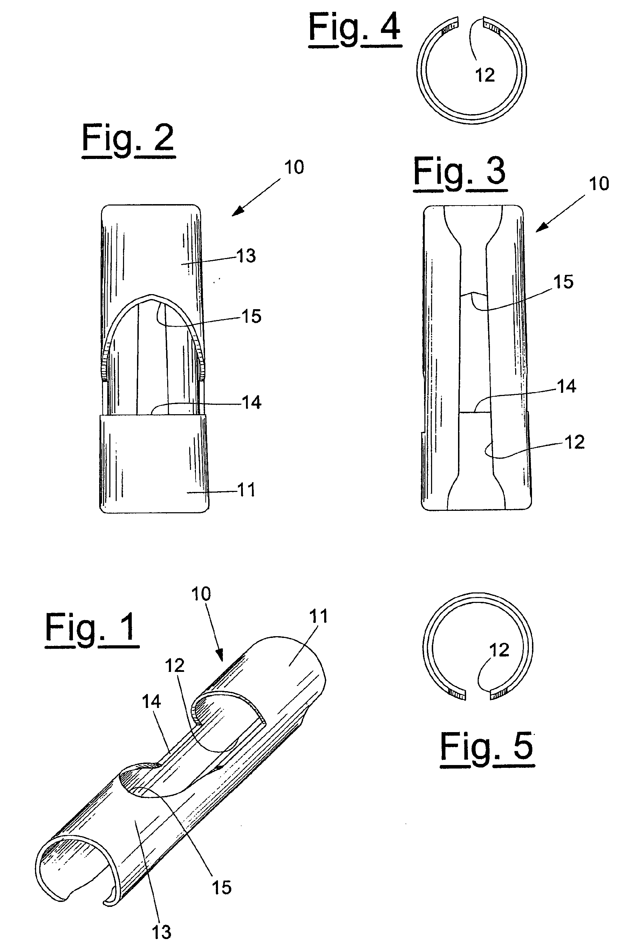

With reference to figure 1, 10 indicates the supporting structure for the application of the method for the lengthening and/or reconstruction of a natural nail according to the present invention, as a whole. It consists of a tubular element 11, for example made of a plastic material, which has a slit 12 along a generating line, said slit broadening out at the ends so as to make it elastically yielding , allowing it to be adapted to all the different dimensions of the user's finger.

Said element 11 also has at one of its ends, a supporting portion 13, for receiving the gel, and an opening or window 14 suitable for completely exposing the natural nail to be treated, said opening 14 being adjacent to the supporting portion 13 of the gel through a rounded edge 15.

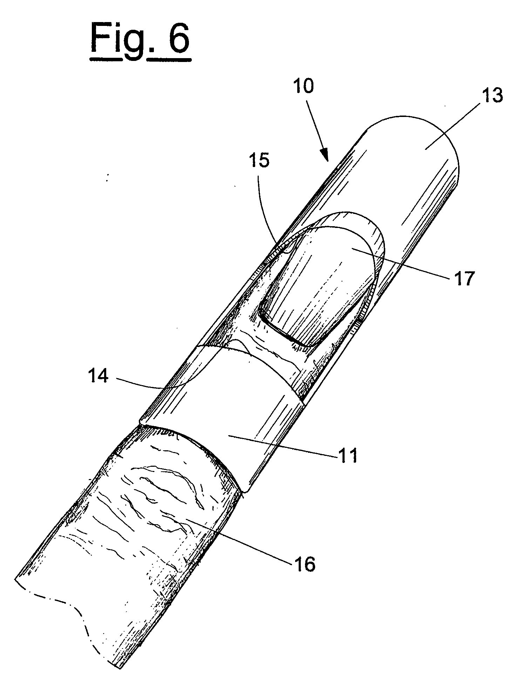

The supporting structure according to the invention is inserted onto the finger 16 of the nail 17 to be treated, as illustrated in figure 6, i.e. with the rounded edge 15 positioned between the lower or underlying surface of the nail 17 and the opposite skin of the finger 16. The method according to the invention can be subsequently applied as described above.

At the end of the treatment, the tubular element 11 can be easily slipped off the finger.

The low viscosity photocuring gel to be used in the reconstruction of nails according to the present invention is packed in a normal glass varnish container, whose surface is preferably metalized or in any case dark-coloured to prevent the gel from hardening due to the effect of sunlight. This guarantees a longer duration of the product.

An object of the present invention also relates to the kit for effecting the method for the lengthening and/or reconstruction of natural nails according to the present invention, said kit comprising a UV lamp, two files, a mousse pumice (abrasive sponge), two low viscosity photocuring gels, a degreasing compound for nails and a gel degreaser.

## Claims

1. Use of a low viscosity photocuring gel consisting of a modified acrylate polymer selected from aliphatic diacrylates modified with a monomeric photo-ionizer, as gel for the lengthening and/or reconstruction of natural nails.

2. The use according to claim 1, **characterized in that** the low viscosity photocuring gel consists of a diacrylates of oxybis(methyl-2,1-ethanediyl).

3. The use according to claim 1, **characterized in that** the photocuring gel has a viscosity at 25°C ranging from 3500 to 4500 cps.

4. The use according to claim 1, **characterized in that** the photocuring gel is diluted with a dispersion of nitrocellulose and titanium dioxide in a mixture of solvents.

5. The use according to claim 4, **characterized in that** the solvents are selected from ethyl acetate, butyl acetate and/or isopropyl alcohol.

6. A method for the lengthening and/or reconstruction of a natural nail which comprises the following phases:
a) preparation of the nail with a mousse pumice (abrasive sponge) and subsequent treatment with a degreasing compound;
b) insertion on the end of the natural nail thus cleaned, of a nail supporting structure;
c) application on the nail, positioned in the nail supporting structure, and optionally on the portion of the supporting structure adjacent to the nail end up to the desired length, of a low viscosity photocuring gel consisting of a modified acrylate polymer selected from aliphatic diacrylates modified with a monomeric photo-ionizer;
d) application to the nail thus treated of a UV radiation for a time varying from 2 to 8 minutes, preferably from 5 to 8 minutes;
e) repetition of phases a)-d) for each natural nail to be lengthened and/or reconstructed and optionally phases c)-d) for each touch-up of the nail already treated;
f) removal of the nail supporting structure from the treated nail;
g) treatment of the nail thus obtained with a degreasing compound of the residual part of the gel after hardening;
h) filing of the gel at the end of the nail until the desired form and length are obtained.

7. The method according to claim 6, **characterized in that** phases c) and d) are carried out at least a second time on the nail itself, before carrying out phase e).

8. The method according to claim 6, **characterized in that** it envisages the application of a tip to the nail to be treated, before effecting phase b).

9. The method according to claim 6, **characterized in that** the photocuring gel used in phase c) is a diacrylate of oxybis(methyl-2,1-ethanediyl).

10. The method according to claim 6, **characterized in that** the photocuring gel used in phase c) has a viscosity at 25°C ranging from 3500 to 4500 cps.

11. The method according to claim 6, **characterized in that** the photocuring gel used in phase c) is diluted with a dispersion of nitrocellulose and titanium dioxide in a mixture of solvents, such as ethyl acetate, butyl acetate and/or isopropyl alcohol.

12. The method according to claim 6, **characterized in that** the degreasing compound in phase a) is a mixture of methyl methacrylate monomer and toluidine.

13. The method according to claim 6, **characterized in that** the degreasing compound in phase g) is isopropyl alcohol.

14. A supporting structure for the application of a gel for the lengthening and/or reconstruction of a natural nail, comprising a tubular element (11) elastically yielding, suitable for containing a finger, said element having a supporting portion (13) of the gel at one end of the structure, and an opening (14) suitable for the complete exposure of the natural nail (17) to be treated, in an adjacent position to said supporting portion (13), said supporting portion (13) having a rounded edge (15) towards the opening (14) suitable for being positioned between the lower surface of the natural nail to be treated (17) and the underlying finger (16).

15. The structure according to claim 14, **characterized in that** said tubular element (11) has a slit (12) along a generating line, making it elastically yielding.

16. The structure according to claim 15, **characterized in that** said slit (12) is enlarged at the ends.

17. A kit for embodying the method for the lengthening and/or reconstruction of natural nails comprising a UV lamp, two files, a mousse pumice (abrasive sponge), two low viscosity photocuring gels, a degreasing compound for nails and a degreasing compound for gels.
